(19) Europäisches Patentamt
European Patent Office
Office européen des brevets

(11) Publication number: 0 279 698 A2

(12) **EUROPEAN PATENT APPLICATION**

(21) Application number: 88301445.8

(22) Date of filing: 19.02.88

(51) Int. Cl.4: C 07 D 493/08
A 01 N 43/90, C 07 D 305/06
//(C07D493/08,319:00,319:00)

(30) Priority: 20.02.87 GB 8703975

(43) Date of publication of application:
24.08.88 Bulletin 88/34

(84) Designated Contracting States:
AT BE CH DE ES FR GB GR IT LI LU NL SE

(71) Applicant: THE WELLCOME FOUNDATION LIMITED
183-193 Euston Road
London NW1 2BP (GB)

THE REGENTS OF THE UNIVERSITY OF CALIFORNIA
2199 Addison Street
Berkeley California 94720 (US)

(72) Inventor: Casida, John Edward
1570 La Vereda Road
Berkeley California 94708 (US)

Palmer, Christopher John
37 Clapgate Lane
Ipswich Suffolk (GB)

Larkin, John Patrick
45 Harrow Road
Leighton Buzzard Bedfordshire (GB)

Smith, Ian Harold
11 Moor End Close
Eaton Bray Bedfordshire (GB)

(74) Representative: Goldin, Douglas Michael et al
J.A. KEMP & CO. 14, South Square Gray's Inn
London WC1R 5EU (GB)

(54) **Pesticidal compounds.**

(57) Bicyclooctane pesticides of the formula

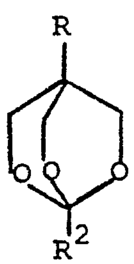

(I)

are prepared by cyclisation of

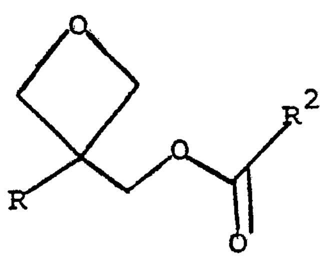

(II)

In these formulae R may be various organic groups and is preferably n-butyl or n-propyl while $R^2$ is a polysubstituted phenyl group.

EP 0 279 698 A2

**Description**

"PESTICIDAL COMPOUNDS"

Acknowledgement of Government Support
This invention was made with US Government support under Grant No. (PO 1 ES 00049) with the National Institute of Health and the University of California. the United States Government has certain rights in this invention.

The present invention relates to novel chemical compounds having pesticidal activity, to methods for their preparation, to compositions containing them and to their use in the control of pests. More particularly the invention relates to a class of heterobicycloalkanes.

The use of certain 2,6,7-trioxabicyclo[2,2,2]octanes is disclosed in European Patent Application No. 152229. It has now been discovered that derivatives of these compounds have interesting pesticidal activity.

Accordingly the present invention provides a compound of the formula (I):

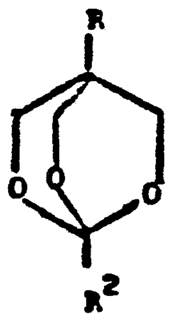

wherein R is $C_{2-10}$ alkyl, alkenyl or alkynyl, each optionally substituted by, or methyl-substituted by, cyano, halo, $C_{3-4}$ cycloalkyl, $C_{1-4}$ alkoxy, $C_{1-4}$ acyloxy, amino, optionally sustituted by one $C_{2-4}$ alkoxycarbonyl or $C_{1-4}$ acyl group or by one or two $C_{1-4}$ alkyl groups, or a group $S(0)_mR^4$ where $R^4$ is $C_{1-4}$ alkyl and m is 0, 1 or 2, or R is $C_{3-10}$ cycloalkyl, $C_{4-10}$ cycloalkenyl or phenyl, each optionally substituted by $C_{1-4}$ alkoxy, $C_{1-3}$ alkyl, $C_{2-4}$ alkenyl, $C_{2-4}$ alkynyl, $C_{1-4}$ acyloxy, halo, cyano,amino optionally substituted by one or two $C_{1-4}$alkoxyacyl, $C_{1-4}$ acyl, $C_{1-4}$ acyloxy, $C_{1-4}$ alkyl or $C_{1-4}$acyloxy optionally substituted by an amino group mono or di-substituted by $C_{1-4}$ alkyl groups or mono-substituted by phenyl optionally substituted by one to five halogen atoms, a group $S(O)_mR^4$ as defined hereinbefore or a group $COR^5$ wherein $R^5$ is $C_{1-4}$ alkyl, $C_{1-4}$ alkoxy or amino optionally substituted by one or two $C_{1-4}$ alkyl groups;

$R^2$ is a phenyl group substituted in at least two of the 3-, 4-, and/or 5-positions, position 4, when substituted, being substituted by halo; amino; nitro; azido; cyano; $C_{1-4}$ haloalkyl; $C_{1-4}$ alkyl; $C_{1-4}$ haloalkoxy; $C_{1-4}$ alkoxy; $S(0)_mR^{4a}$, wherein $R^{4a}$ is a group $R^4$ as defined hereinbefore; or $C_{1-4}$ alkyl substituted by one to three fluorine atoms; or by a group $COR^5$ as defined hereinbefore; and at the 3- and/or 5-position at the phenyl ring when substituted, being substituted by a halo, $C_{1-4}$ haloalkyl, $C_{1-4}$ alkyl, $C_{1-4}$ haloalkoxy, $C_{1-4}$ alkoxy, $C_{1-4}$ alkyl substituted by one three fluorine atoms, $S(0)_mR^4$ as defined hereinbefore, nitro, azido or cyano group by a group $COR^5$ as defined hereinbefore or by a group of the formula $C \equiv CR^6$ wherein $R^6$ is hydrogen, or a silyl group substituted by three $C_{1-4}$ alkyl groups or two $C_{1-4}$ alkyl groups and a phenyl group; and further optionally substituted at the 2- and/or 6-position by fluoro, chloro except that:

1. when R is tert.butyl, then $R^2$ may not be 3,4-dichlorophenyl or pentafluorophenyl;
2. when R is n-propyl, then $R^2$ may not be 3-chloro-4-iodophenyl or 4-iodo-3-nitrophenyl;
3. when R is cyclohexyl, then $R^2$ may not be 3,4-dichlorophenyl;
4. when R is isopropyl, then $R^2$ may not be pentafluorophenyl.

Suitably R is propyl, butyl, pentyl, $C_{2-5}$alkenyl or alkynyl, $C_{3-7}$ cycloalkyl or phenyl each optionally substituted by fluoro, chloro or bromo. Most suitably R is n-propyl, n-butyl, i-butyl, sec-butyl, t-butyl, cyclopentyl or cyclohexyl and preferably R is n-propyl, n-butyl, i-butyl, t-butyl or cyclohexyl.

Suitably when the substituents on the phenyl group in $R^2$ are at the 3-, 4- and/or 5-positions these substituents include fluoro, chloro, bromo, iodo, cyano, azido, $C_{1-3}$ alkyl or $C_{1-3}$ alkoxy each optionally substituted by halo, or $C_{2-3}$ alkynyl optionally substituted by a silyl group substituted by three $C_{1-4}$alkyl groups or two $C_{1-4}$ alkyl groups and a phenyl group. The halo substituent can be fluoro, chloro, bromo or iodo. Suitable haloalkyl substituents are polyhalomethyl or polyhaloethyl, the halogen preferably being fluorine. Most suitably the preferred haloalkyl substituent is trifluoromethyl.

Preferably the phenyl group in $R^2$ is substituted by three substituents in the 3-, 4- and 5-positions or by two substituents in the 3- and 4-positions.

The present invention also encompasses radioisotopes of compounds of formula (I) particularly those in which one carbon atom is $C^{13}$ or $C^{14}$, or one to three hydrogen atoms are replaced by tritium.

Preferred compounds of the present invention include:

1. 1-(3-cyano-4-methylphenyl)-4-tert.butyl-2,6,7-trioxabicyclo[2.2.2]octane.
2. 1-(3,4,5-trichlorophenyl)-4-tert.butyl-2,6,7-trioxabicyclo[2.2.2]octane.
3. 1-(3-methoxy-4-chlorophenyl)-4-tert.butyl-2,6,7-trioxabicyclo[2.2.2]octane.
4. 1-(4-iodo-3,5-dichlorophenyl)-4-tert.butyl-2,6,7-trioxabicyclo[2.2.2]octane.
5. 1-(3-cyano-4-chlorophenyl)-4-tert.butyl-2,6,7-trioxabicyclo[2.2.2]octane.
6. 1-(3-cyano-4-methoxyphenyl)-4-tert.butyl-2,6,7-trioxabicyclo[2.2.2]octane.
7. 1-(4-azido-3,5-dichlorophenyl)-4-tert.butyl-2,6,7-trioxabicyclo[2.2.2]octane.
8. 1-(4-cyano-3,5-dichlorophenyl)-4-tert.butyl-2,6,7-trioxabicyclo[2.2.2]octane.
9. 1-(4-bromo-3,5-dichlorophenyl)-4-tert.butyl-2,6,7-trioxabicyclo[2.2.2]octane.
10. 1-(3,4,5-tri-iodophenyl)-4-tert.butyl-2,6,7-trioxabicyclo[2.2.2]octane.
11. 1-(2-chloro-4-cyanophenyl)-4-tert.butyl-2,6,7-trioxabicyclo[2.2.2]octane.
12. 1-(4-bromo-3-chlorophenyl)-4-n-propyl-2,6,7-trioxabicyclo[2.2.2]octane.
13. 1-(4-bromo-3,5-dichlorophenyl)-4-n-propyl-2,6,7-trioxabicyclo[2.2.2]octane.
14. 1-(4-chloro-3-trifluoromethylphenyl)-4-n-butyl-2,6,7-trioxabicyclo[2.2.2]octane.
15. 1-(3-fluoro-4-iodophenyl)-4-n-propyl-2,6,7-trioxabicyclo[2.2.2]octane.

In a further aspect, the present invention provides a process for the preparation of a compound of the formula (I). The process for the preparation of a compound of the formula (I) may be any method known in the art for preparing analogous compounds, for example:

By the cyclisation of a compound of the formula (II):

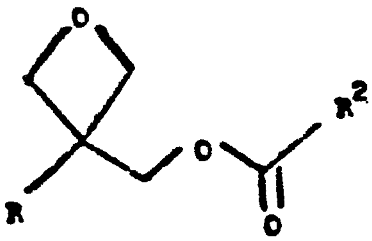

(II)

wherein R and $R^2$ are as hereinbefore defined in the presence of an acid catalyst. Boron trifluoride etherate is a particularly preferred acid catalyst for this cyclisation which will normally be carried out in an inert solvent, such as a halogenated hydrocarbon, conveniently dichloromethane, at below ambient temperature, for example between -100 and 0°C and conveniently between -70 and -50°C.

Compounds where $R^2$ contains a -C≡CR$^6$ substituent can be obtained by the reaction of the corresponding compound (obtained by cyclisation of II) which contains iodo in place of -C≡C-R$^6$ with a compound HC≡CR$^6$ wherein $R^6$ is as hereinbefore defined. This reaction is carried out in the presence of a suitable palladium catalyst well-known to those skilled in the art for this type of reaction, for example bis-triphenylphosphine palladium dichloride, and a catalytic amount of a cuprous halide, such as cuprous iodide. The reaction will normally be carried out in the presence of basic solvent such as diethylamine or triethylamine at a non-extreme temperature, for example between -50° and 100°C and conveniently at room temperature.

The compounds of formula II are themselves new compounds that form a further aspect of the present invention.

The compounds of the formula (II) may be prepared by the reaction of compounds of the formula (III) and (IV):

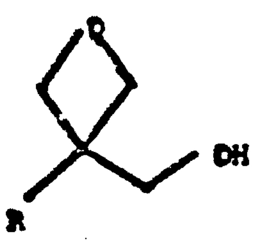

(III) $R^2$-C(=O)L (IV)

wherein R and $R^2$ are as hereinbefore defined and L is a leaving group such as halo. This reaction conveniently takes place in an inert solvent in the presence of base at a non-extreme temperature. Halogenated hydrocarbons, such as dichloromethane are particularly suitable solvents, pyridine is a preferred base and the reaction will conveniently be carried out at between -50 and 100°C, preferably at 0°C.

The compounds of the formula (III) may in turn be prepared from compounds of the formula (V):

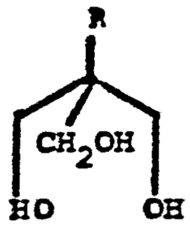

(V)

wherein R is as defined above by reaction with diethyl carbonate in the presence of a strong base, for example potassium hydroxide, in a polar solvent, such as an alcohol,for example ethanol, at an elevated temperature, for example between 50 and 100° C.

The triol of the formula (V) may be prepared:

(i) by the reaction of an aldehyde $RCH_2CHO$ with formaldehyde. This reaction is normally carried out in a base, such as a metal hydroxide in a polar solvent, for example water, at a non-extreme temperature, for example between 0 and 120° C.

(ii) In certain cases, it may be convenient to prepare triol derivatives where one of the hydroxy groups is protected, by reduction of an ester of the formula (VI):

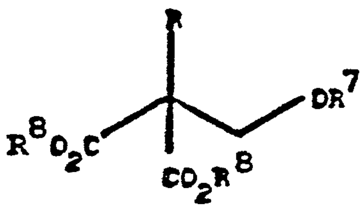

(VI)

wherein $R^7$ is a hydroxy protecting group such as benzyl and $R^8$ is $C_{1-4}$ alkyl. This reduction is suitably carried out by a complex hydride such as lithium aluminium hydride in an inert solvent conveniently an ether. The compound of the formula (VI) may be prepared from the corresponding compound $RCH(CO_2R^8)_2$ by reaction with a compound $XCH_2OR^7$, wherein X is a leaving group such as a halo, in the presence of a strong base, such as sodium hydride.

Compounds of formula (I) which contain iodo substituents in the phenyl ring $R^2$ may also be prepared from the corresponding bromo compound. This reaction proceeds by the reaction of the bromo compound with an alkyl-lithium compound, for example n-butyl-lithium, in an inert solvent, such as an ether, conveniently diethyl ether, at a non-extreme temperature, for example between -80° and 20° C and conveniently between -70 and 0° C, to give the corresponding lithium intermediate which is then reacted with iodine in an inert solvent such as an ether, conveniently diethyl ether. The reaction sequence is conveniently carried out in situ, the organo-lithium intermediate not being isolated.

The bromo compound may be prepared by the cyclisation of a compound of the formula (II) wherein $R^2$ is a bromo-substituted phenyl group under the conditions previously described for the corresponding iodo compound.

The bromophenyl compound and polysubstituted phenyl compounds of the invention may be prepared by the reaction of a compound analogous to that of the formula (V) and wherein R is as defined in relation to formula (I) with an orthocarboxylate of the formula $R^{2x}$ $C(OR^9)_3$ wherein $R^{2x}$ is a bromo-substituted phenyl group or group $R^2$ and $R^9$ is $C_{1-4}$ alkyl, phenyl or $C_{7-8}$ aralkyl. Suitably $R^9$ is methyl or ethyl, preferably methyl. The reaction is normally carried out in the presence of an acid such as a mineral acid, conveniently hydrochloric acid or a sulphonic acid derivative, such as toluene sulphonic acid, or an acid resin, or in the presence of a trialkylamine, such as triethylamine, at an elevated temperature, for example between 50 and 200° C, conveniently between 120 and 170° C. The reaction may conveniently be carried out in the absence of a solvent but a suitable solvent may be added if desired. The preparation of such bromophenyl substituted bicyclooctanes is described in published European Patent Application No. 152229 and in our copending European Application No. 86305820.2.

(iv) Compounds of the formula (I) in which the phenyl ring $R^2$ contains a $-C{\equiv}-R^6$ group wherein $R^6$ is hydrogen may also be prepared by dehydrobromination of the corresponding 1,2-bromoethyl compound, i.e. a compound wherein $R^2$ is (1,2-bromoethyl)phenyl. This reaction is conveniently carried out in the presence of sodamide in liquid ammonia. The reaction may be carried out in an inert solvent, such as an ether, for example tetrahydrofuran at a non-extreme temperature, for example between -20 and 50° C and conveniently at room temperature.

(v) It is often convenient to prepare compounds of the formula (I) by interconversion from other compounds of the formula (I), for example:

(ii) when it is desired to prepare a compound of the formula (I) wherein $R^6$ is hydrogen by the desilylation of a compound of the formula (I) wherein $R^6$ is a tri-$C_{1-4}$ alkylsilyl group. This reaction may be carried out by methods well-known to those skilled in the art, for example by reaction with tetrabutyl-ammonium fluoride in an ether, such as tetrahydrofuran, at a non-extreme temperature, for example between 0° and 70° C and conveniently at room temperature.

The compounds of formula (I) may be used to control arthropods such as insect and acarine pests. Thus, the present invention provides a method for the control of arthropods which comprises administering to the arthropod or to an environment susceptible to arthropod attack an arthropodically effective amount of a compound of the formula (I). The present invention also provides a method for the control and/or eradication of arthropod infestations on animals (including humans) which comprises administering to the animal an effective amount of a compound of the formula (I). The present invention further provides for the compounds of the formula (I) for use in human and veterinary medicine for the control of arthropod pests.

The compounds of formula (I) may be used for such purposes by application of the compounds themselves or in diluted form in known fashion as a dip, spray, lacquer, foam, dust, powder, aqueous suspension, paste, gel, shampoo, grease, combustible solid, vapourising mat, combustible coil, bait, wettable powder, granule, aerosol, emulsifiable concentrate, oil suspensions, oil solutions, pressure-pack, impregnated article or pour-on formulation. Dip concentrates are not applied per se, but diluted with water and the animals immersed in a dipping bath containing the dip wash. Sprays may be applied by hand or by means of a spray race or arch. The animal, plant or surface being treated may be saturated with the spray by means of high volume application or superficially coated with the spray by means of light or ultra low volume application. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material against which animals rub transferring the material to their skins. Pour-on formulations are dispensed as a unit of liquid of small volume on to the backs of animals such that all or most of the liquid is retained on the animals.

The compounds of formula (I) may be formulated either as formulations ready for use on the animals, plants or surface or as formulations requiring dilution prior to application, but both types of formulation comprise a compound of formula (I) in intimate admixture with one or more carriers or diluents. The carriers may be liquid, solid or gaseous or comprise mixtures of such substances, and the compound of formula (I) may be present in a concentration of from 0.025 to 99% w/v depending upon whether the formulation requires further dilution.

Dusts, powder and granules comprise the compound of formula (I) in intimate admixture with a powdered solid inert carrier for example suitable clays, kaolin, talc, mica, chalk, gypsum, vegetable carriers, starch and diatomaceous earths.

Sprays of a compound of formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 90% w/v of the composition and may be selected from kerosene, ketones, alcohols, xylene, aromatic naphtha, and other solvents known in the formulating art. The concentration of emulsifiers may be varied within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates.

Wettable powders comprise an inert solid carrier, one or more surface-active agents, and optionally stabilisers and/or anti-oxidants.

Emulsifiable concentrates comprise emulsifying agents, and often an organic solvent, such as kerosene, ketones,alcohols, xylenes, aromatic naphtha, and other solvents known in the art.

Wettable powders and emulsifiable concentrates will normally contain from 5 to 95% by weight of the active ingredient, and are diluted,for example with water, before use.

Lacquers comprise a solution of the active ingredient in an organic solvent, together with a resin, and optionally a plasticiser.

Dip washes may be prepared not only from emulsifiable concentrates but also from wettable powders, soap-based dips and aqueous suspensions comprising a compound of formula (I) in intimate admixture with a dispersing agent and one or more surface-active agents.

Aqueous suspensions of a compound of formula (I) may comprise a suspension in water together with suspending, stabilising or other agents. The suspensions or solutions may be applied per se or in a diluted form in known fashion.

Greases (or ointments) may be prepared from vegetable oils, synthetic esters of fatty acids or wool fat together with an inert base such as soft paraffin. A compound of formula (I) is preferably distributed uniformly through the mixture in solution or suspension. Greases may also be made from emulsifiable concentrates by diluting them with an ointment base.

Pastes and shampoos are also semi-solid preparations in which a compound of formula (I) may be present as a uniform dispersion in a suitable base such as soft or liquid paraffin or made on a non-greasy basis with glycerin, mucilage or a suitable soap. As greases, shampoos and pastes are usually applied without further

dilution, they should contain the appropriate percentage of the compound of formula (I) required for treatment.

Aerosol sprays may be prepared as a simple solution of the active ingredient in the aerosol propellant and co-solvent such as halogenated alkanes and the solvents referred to above, respectively. Pour-on formulations may be made as a solution or suspension of a compound of formula (I) in a liquid medium. An avian or mammal host may also be protected against infestation of acarine ectoparasites by means of carrying a suitably-moulded, shaped plastics article impregnated with a compound of formula (I). Such articles include impregnated collars, tags, bands, sheets and strips suitably attached to appropriate parts of the body. Suitably the plastics material is a polyvinyl chloride (PVC).

The concentration of the compound of formula (I) to be applied to an animal will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the formulation but in general will lie in the range of from 0.0001% to 0.5% except for undiluted formulations such as pour-on formulations which in general will be deposited at a concentration in the range from 0.1 to 20.0% and preferably 0.1 to 10%.

The compounds of formula (I) are also of use in the protection and treatment of plant species, in which case an effective insecticidal or acaricidal amount of the active ingredient is applied. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and 1kg/ha. Typical formulations for agricultural use contain between 0.0001% and 50% of a compound of formula (I) and conveniently between 0.l and 15% by weight of a compound of the formula (I).

Particular crops include cotton, wheat, maize, rice, sorghum, soya, vines, tomatoes, potatoes, fruit trees and spruce.

Dusts, greases, pastes and aerosol formulations are usually applied in a random fashion as described above and concentrations of 0.001 to 20% w/v of a compound of formula (I) in the applied formulation may be used.

The compounds of formula (I) have been found to have activity against the common housefly (Musca domestica). In addition, certain compounds of formula (I) have activity against other arthropod pests including Tetranychus urticae, Plutella xylostella, Myzus spp., Culex spp. and Blattella germanica). The compounds of formula (I) are thus useful in the control of arthropods e.g. insects and acarines in any environment where these constitute pests, e.g. in agriculture, in animal husbandry, in public health control and in domestic situations.

Insect pests include members of the orders Coleoptera (e.g. Anobium, Tribolium, Sitophilus, Diabrotica, Anthonomus or Anthrenus spp.), Lepidoptera (e.g. Ephestia, Plutella, Chilo, Heliothis, Spodoptera or Tineola spp.), Diptera (e.g. Musca, Aedes, Culex, Glossina, Stomoxys, Haematobia, Tabanus, Hydrotaea, Lucilia, Chrysomia, Callitroga, Dermatobia, Hypoderma, Liromyza and Melophagus spp.), Phthiraptera (Malophaga, e.g. Damalina spp. and Anoplura e.g. Linogathus an Haematopinus spp.), Hemiptera (e.g. Aphis, Bemisia, Myzus spp., Aleurodes, Nilopavata, Nephrotetix or Cimex spp.), Orthoptera (e.g. Schistocerca or Acheta spp.), Dictyoptera (e.g. Blattella, Periplaneta or Blatta spp.), Hymenoptera (e.g. Siphonaptera (e.g. Ctenocephalides or Pulex spp.), Thysanura (e.g. Lepisma spp.), Dermaptera (e.g. Forficula spp.) and Pscoptera (e.g. Peripsocus spp.).

Acarine pests include ticks, e.g members of the genera Boophilus, Rhipicephalus, Amblyomma, Hyalomma, Ixodes, Haemaphysalis, Dermocentor and Anocentor, and mites and manges such as Tetranychus, Psoroptes, Notoednes, Psorergates, Chorioptes and Demodex spp.

Compounds of the invention may be combined with one or more other active ingredients (for example pyrethroids, carbamates and organophosphates) and/or with attractants and the like. Furthermore, it has been found that the activity of the compounds of the invention may be enhanced by the addition of a synergist or potentiator, for example: one of the oxidase inhibitor class of synergists, such as piperonyl butoxide or propyl 2-propynylphenylphosphonate; a second compound of the invention; or a pyrethroid pesticidal compound. When an oxidase inhibitor synergist is present in a formula of the invention, the ratio of synergist to compound of formula (I) will be in the range 25:1 - 1:25 e.g. about 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin) and organic or inorganic bases e.g. trialkylamines such as triethylamine which can act as basic stabilisers and as scavengers.

The following Examples illustrate, in a non-limiting manner, preferred aspects of the invention. All temperatures are in degrees Celsius.

General Synthetic Methods and Procedures:

Various compounds were synthesised and characterised in accordance with the following experimental procedures.

$^1H$ N.m.r. spectra were obtained on a Bruker AM-250 or WM-300 spectrometers in deuteriochloroform solutions with tetramethylsilane as internal standard.

Mass spectra were obtained on a Finnigan 4500 or Hewlett Packard 5985B instruments.

Example 1

1-(4-Chloro-3-trifluoromethylphenyl)-4-n-propyl-2,6,7-trioxabicyclo[2.2.2]octane

(i) To a stirred mixture of n-valeraldehyde (172 g) and water (2 l.) was added solid calcium hydroxide (112 g) and formaldehyde solution (1.4 l. of 40% aqueous solution). The reaction temperature was maintained below 40° and the addition took about 45 minutes. The mixture was then maintained at 60° for 5 hours. The reaction mixture was filtered through Kieselguhr and the filtrates were evaporated in vacuo. The residue was treated with hot methanol (2 l.) and the mixture was filtered through Kieselguhr. The filtrates were evaporated in vacuo A viscous oily product was obtained (458 g) and was purified as follows:-

A solution of the crude product and acetic acid (200 ml) was stirred at room temperature. Acetic anhydride (1.2 l.) was added over 4 hours. The temperature rose to 65°. Stirring was continued for 12 hours. The reaction mixture was added over 3 hours to cold water (3 l.) with stirring. Stirring was continued for 3 hours. The aqueous mixture was extracted with diethyl ether. The ether extracts were washed with aqueous sodium hydrogen carbonate solution and then with brine. The extracts were dried over anhydrous magnesium sulphate and then evaporated in vacuo.

Distillation gave 2,2-di-(hydroxymethyl)pentan-1-ol triacetate (238 g), a colourless oil (b.p. 120-140°, 1.5 mm).

Nuclear magnetic resonance spectrum (NMR) was as follows: $^1H$ (ppm from TMS in $CDCl_3$, integral, number of peaks): 1.00, 3H,m; 1.40, 4H,m; 2.10, 9H,s; 4.00, 6H,s.

Sodium (0.5 g) was added to a stirred solution of the above triacetate (238 g) in methanol (2.5 l.). The mixture was refluxed, with stirring, for 72 hours. The mixture was evaporated in vacuo.

2,2-di-(hydroxymethyl)pentan-1-ol (87 g) was obtained as colourless crystals (m.pt. 93°).

Ref. W.E. Conrad, L.A. Levasseur, R.F. Murphy, N.L. Hare and H.E. Conrad, J. Org. Chem, 1962, 27, 2227.

(ii) A mixture of 2,2-di-(hydroxymethyl)pentan-1-ol (24.6 g), diethyl carbonate (20.1 ml), potassium hydroxide (0.3 g) and dry ethanol (2 ml) was heated to gentle reflux (oil bath 110-120°) under a stream of nitrogen for 30 minutes. After this time the ethanol formed was removed by distillation at atmospheric pressure (oil bath 130-140°, still head temperature 76°). The pressure was reduced to 20 mm.Hg and the oil bath temperature adjusted to 230°. 3-Hydroxymethyl-3-n-propyl oxetane distilled as a colourless liquid (16.7 gms, head temperature 120-126°).

Gas-liquid chromatography (g.l.c.): OV-210 at 120° produced one peak. Nuclear magnetic resonance spectrum (N.M.R.) was as follows: $^1H$ (ppm from TMS in $CDCl_3$, integral, multiplicity): 0.70-1.80, 7H,m; 3.60, 2H,m; 4.35, 4H,s.

(iii) a) Diazonium salt preparation

Sodium nitrite (9.75 g) in water (30 ml) was added gradually to a stirred mixture of 5-amino-2-chlorobenzotrifluoride (25 g supplied by Aldrich), concentrated hydrochloric acid (32 ml) and crushed ice. The mixture was stirred at 0° for 30 minutes. A neutral pH was achieved by addition of solid sodium carbonate (15 g).

b) Copper (1) cyanide preparation

Copper (1) chloride (16 g) was suspended in distilled water (100 ml), sodium cyanide (19 g) was added slowly to the stirred suspension. The mixture was then cooled and benzene (150 ml) was added.

The copper (1) cyanide solution was stirred vigorously and the cold diazonium salt solution was added gradually over 30 minutes, maintaining the resulting reaction mixture at 0°. The reaction mixture was stirred at 0° for 30 minutes and the temperature was allowed to rise to 20° slowly. The mixture stood at 20° for 12 hours and was then heated at 50° for 15 minutes. The mixture was cooled and the organic layer was separated and steam-distilled for 4 hours. The distillate was extracted with benzene. The organic extracts were dried over anhydrous magnesium sulphate and the solvent was removed in vacuo. The resulting oil (10.4 g) was purified by chromatography on silica, eluting with 1:10 ethyl acetate:hexane. 2-Chloro-5-cyanobenzotrifluoride was obtained as a yellow solid (5.4 g, mpt 61°).

Nuclear magnetic resonance spectrum (N.M.R.) was as follows: $^1H$ (p.p.m. from TMS in $CDCl_3$, integral, number of peaks $J_{Hz}$): 7.65, 1H, d, 6; 7.75, 1H, d, 6; 8.05, 1H,s.

Infrared spectrum (IR) nujol mull): $_{max}$ 2265 cm$^{-1}$.
Mass spectrum: Chemical Ionisation M+1 206.

(iv) A mixture of 2-chloro-5-cyanobenzotrifluoride (0.5 g), 50% aqueous sulphuric acid (6 ml) and acetic acid (4 ml) was refluxed for 90 minutes. The mixture was poured into crushed ice and the aqueous mixture was extracted with diethyl ether. The ethereal extracts were extracted with 5% aqueous sodium carbonate solution. The aqueous alkaline extracts were acidified with 2N hydrochloric acid solution. The aqueous acidic mixture was extracted with diethyl ether. The ethereal extracts were dried over anhydrous magnesium sulphate. The solvent was removed in vacuo 4-Chloro-3-trifluoromethylbenzoic acid was obtained as a colourless solid (0.5 g, mpt 159°).

Nuclear magnetic resonance spectrum (N.M.R.) was as follows: $^1H$ (p.p.m. from TMS in $(CD_3)_2SO$, integral, number of peaks, $J_{Hz}$): 7.50, 1H, d, 6; 8.00, 2H,m.

Infrared spectrum (1R) (nujol mull): $_{max}$ 2650 (strong and broad), 1705 (s) cm$^{-1}$.
Mass spectrum: Chemical Ionisation M+1 225.

(v) A mixture of 4-chloro-3-trifluoromethylbenzoic acid (4.3 g), thionyl chloride (2.9 ml) and dry benzene (50 ml) was refluxed for 3 hours. The reaction mixture was cooled and the solvent was removed in vacuo 4-Chloro-3-trifluoromethylbenzoyl chloride was obtained as a waxy solid (4.5 g).

(vi) 4-Chloro-3-trifluoromethylbenzoyl chloride (2.8g.) in dry diethyl ether (75 ml.) was added to a stirred solution of 3-hydroxymethyl-3-n-propyloxetane (1.5g.) and pyridine (2.0 ml.) in diethyl ether (75 ml.) The mixture was stirred at room temperature for 6 hours and poured into water. The aqueous mixture was extracted with diethyl ether and the ethereal extracts were washed with water and dried over anhydrous magnesium sulphate. The ethereal solution was evaporated in vacuo The residue was purified by chromatography on silica pre-eluted with 1% triethylamine in hexane.

3-(4-Chloro-3-trifluoromethylbenzoyloxymethyl)-3-n-propyloxetane was obtained as a colourless oil (1.9g.) Nuclear magnetic resonance spectrum (NMR) was as follows: $^1H$(p.p.m. from TMS in $CDCl_3$, integral, number of peaks, $J_{Hz}$): 0.80-2.20, 7H,m; 4.60, 6H,m; 7.40, 1H,d,7; 8.10 ,2H,m.

Infrared spectrum (IR) (liquid film): 1735 $cm.^{-1}$
Mass Spectrum: Chemical Ionisation M + 1 337.

(vii) Boron trifluoride etherate (0.15 ml.) was added to a stirred solution of 3-(4-chloro-3-trifluoromethylbenzoyloxymethyl)-3-n-propyloxetane (1.7g.) in dry dichloromethane (25 ml.) at -70°, under a current of dry nitrogen. The stirred mixture was allowed to warm up to room temperature over a period of 6 hours. Triethylamine (1.5 ml.) was added and the mixture was poured into water. The aqueous mixture was extracted with dichloromethane. The extracts were washed with water and dried over anhydrous magnesium sulphate and the solvent was removed in vacuo The residue was purified by chromatography on alumina (alumina Woelm TSC) eluting with 1:4 dichloromethane : hexane, saturated with ammonia.

1-(4-Chloro-3-trifluoromethylphenyl)-4-n-propyl-2,6,7-trioxabicyclo[2.2.2]octane (compound 1) was obtained as a colourless solid (1.06g. mpt. 65-68°).

Following the above procedure and using the appropriate benzoyl chloride and 3-substituted-3-hydroxymethyloxetane the 1-aryl bicyclo-octanes 2-9, 13-25, 27 and 28 (see Table I) were prepared.

TABLE I

1-Arylbicyclooctanes

| Compound | R | X | Synthetic Method Example |
|---|---|---|---|
| 1 | n-Pr | 3-$CF_3$-4-Cl | 1 |
| 2 | n-Pr | 3-Cl-4-Br | 1 |
| 3 | n-Pr | 3,5-di-Cl-4-Br | 1 |
| 4 | n-Pr | 3,5-di-Cl | 1 |
| 5 | n-Pr | 3,5-di-$CF_3$ | 1 |
| 6 | n-Pr | 3,5-di-Cl-4-$NH_2$ | 1 |
| 7 | n-Bu | 3-$CF_3$-4-Cl | 1 |
| 8 | n-Pr | 2,3,5,6-tetra-F-4-Br | 1 |
| 9 | n-Pr | 3-I-4-Cl | 1 |
| 10 | n-Pr | 3-C≡$CSiMe_3$-4-Cl | 3 |
| 11 | n-Pr | 3-C≡CH-4-Cl | 3 |

TABLE I (Continued)

1-Arylbicyclooctanes

| Compound | R | X | Synthetic Method Example |
|---|---|---|---|
| 12 | n-Pr | 2,3,5,6-tetra-F-4-n-Bu | 4 |
| 13 | n-Pr | 2,3,4,5,6-penta-F | 1 |
| 14 | n-Pr | 3,4-di-iodo | 1 |
| 15 | t-Bu | 3-CN-4-Me | 1 |
| 16 | t-Bu | 3,4,5-tri-Cl | 1 |
| 17 | t-Bu | 3-MeO-4-Cl | 1 |
| 18 | t-Bu | 3,5-di-Cl-4-I | 1 |
| 19 | t-Bu | 3-CN-4-Cl | 1 |
| 20 | t-Bu | 3-CN-4-MeO | 1 |
| 21 | t-Bu | 3,5-di-Cl-4-$N_3$ | 1 |
| 22 | t-Bu | 3,5-di-Cl-4-CN | 1 |

TABLE I (Continued)

1-Arylbicyclooctanes

| Compound | R | X | Synthetic Method Example |
|---|---|---|---|
| 23 | t-Bu | 3,5-di-Cl-4-Br | 1 |
| 24 | t-Bu | 3,4,5-tri-I | 1 |
| 25 | t-Bu | 2-Cl-4-CN | 1 |
| 26 | t-Bu | 3-Cl-4-MeO | 2 |
| 27 | t-Bu | 3,4-di-F | 1 |
| 28 | n-Pr | 3-F-4-I | 1 |

Characterising data for these compounds are set out in Table II below.

TABLE II

| Compound | mpt | Mass Spectrum Chemical Ionisation M + 1 | Infrared Spectrum | Nuclear Magnetic Resonance Spectra: $^1H$ carried out in $CDCl_3$ and expressed as p.p.m. downfield from TMS, (number of protons, multiplicity, $J_{Hz}$) |
|---|---|---|---|---|
| 1 | 65-68° | 337 | 1180(s),1145(s)1120(s) 1045(s),1020(s) | 0.70-1.20,7H,m; 4.00,6H,s; 7.35,1H, d,7; 7.50-7.90,2H, m. |
| 2 | 88-89° | 347 349 | 1020(s),1010(s) | 0.85-1.35,7H,m; 4.10,6H,s; 7.30-7.80,3H,m. |
| 3 | solid | 381 383 | 1020(s),1000(s) | 0.90-1.40,7H,m; 4.10,6H,s; 7.60,2H,s |
| 4 | solid | 303 | | 0.80-1.40,7H,m; 4.10,6H,s; 7.35,1H,m; 7.50,2H,m. |
| 5 | solid | 371 | | 0.80-1.40,7H,m; 4.10,6H,s; 7.90,1H,m; 8.10,2H,m |
| 6 | 132.4° | 318 | 3480(m),3350(m) 1019(s),1000(s) 960(s) | 0.85-1.35,7H,m; 4.20,6H,s; 4.50,2H,s; 7.50,2H,s |
| 7 | solid | 351 | | 0.75-1.40,7H,m; 4.10,6H,s; 7.30-8.00,3H,m |
| 8 | 112-113° | 385 | | 0.90 3H,t, 7; 1.40, 4H,m; 4.10,6H,s. |
| 9 | 106.5° | 395 | | 0.95, 3H, m; 1.25, 4H, m; 4.10,6H, s; 7.40, 1H, d, 7; 7.55, H, dd, 7 and 1.5; 8.12, 1H, d, 1.5 |

TABLE II (Continued)

| | | | |
|---|---|---|---|
| 10 | 75° | 365 | 0.25, 9H, s; 1.00, 3H, m; 1.20, 4H, m; 4.10, 6H, s; 7.40, 2H, m; 7.80, 1H, m |
| 11 | 141° | 293 | 0.95, 3H, m; 1.25, 4H, m; 3.35, 1H, s; 4.10, 6H, s; 7.40, 1H, d, 7; 7.55, 1H, dd, 7 and 1.5; 7.85, 1H, d, 1.5 |
| 12 | 122-123° | 363 | 0.60-1.60, 14H,m; 2.55, 2H, t, 7; 4.00, 6H, s |
| 13 | 113-114° | 325 | 0.80-1.60, 7H, m; 4.00, 6H, s |
| 14 | 129.5° | 487 | 0.95, 3H, m; 1.25, 4H, m; 4.10, 6H, s; 7.32, 2H, dd, 7 and 2; 7.82, 1H, d, 7; 8.10, 1H, d, 2 |
| 15 | 215-216° | 288 | 0.90, 9H, s; 2.50, 3H, s; 4.15, 6H, s; 7.25, 1H, d; 7.65, 1H, dd; 7.80, 1H, d |
| 16 | 160-162° | 351 | 0.90, 9H, s; 4.15, 6H,s; 7.60, 2H, s |
| 17 | 164-166° | 313 | 0.90,9H, s; 3.90, 3H,s; 4.15, 6H, s; 7.10, 1H, dd; 7.15, 1H, d; 7.30, 1H, d |
| 18 | 185-187° | 443 | 0.90, 9H,s; 4.15, 6H, s; 7.55, 2H, s |
| 19 | 144-145° | 308 | 0.90 9H, s; 4.15,6H, s; 7.45, 1H, d; 7.70, 1H, dd; 7.90, 1H, d |

TABLE II (Continued)

| | | | |
|---|---|---|---|
| 20 | 214-215° | 304 | 0.90, 9H, s; 3.90, 3H, s; 4.15, 6H, s; 6.90, 1H, d; 7.75, 1H, dd; 7.80, 1H, d |
| 21 | 124-126° Dec. | 358 | 0.90,9H, s; 4.15,6H, s; 7.50, 2H, s |
| 22 | 188-189° | 342 | 0.90, 9H, s; 4.15,6H, s; 7.65, 2H, s |
| 23 | 187-189° | 395 | 0.90, 9H, s; 4.15, 6H, s; 7.70, 2H, s |
| 24 | 194-196° | 627 | 0.90, 9H, s; 4.10, 6H, s; 8.00, 2H, s |
| 25 | 203-205° | 308 | 0.90, 9H,s; 4.15, 6H, s; 7.50, 1H, dd; 7.65, 1H, d; 7.85, 1H, d |
| 26 | 217-219° | 313 | 0.90, 9H,s; 3.85, 3H, s; 4.15, 6H, s; 6.85, 1H, d; 7.45, 1H, dd; 7.60, 1H, d |
| 27 | 157-158° | 285 | 0.90, 9H, s; 4.15, 6H, s; 7.05-7.50, 3H, m; 7.15, 1H, dd; 7.32, 1H, dd. |
| 28 | Solid | 379 | 0.90, 3H, t, 7; 1.25, 4H, m; 4.10, 6H, s; 7.15, 1H, 7.32, 1H, 7.72, 1H |

Characterising data for the intermediate oxetane ester precursors is set out in Table III below.

TABLE III

CHARACTERISING DATA FOR OXETANE ESTER PRECURSORS

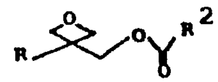

| Compound | R | $R^2$ | | Mass Spectrum Chemical | Infrared Spectrum | Nuclear Magnetic Resonance Spectra: $^1H$ carried out in $CDCL_3$ and expressed as p.p.m. downfield from TMS (number of protons of multiplicity, $J_{Hz}$) |
|---|---|---|---|---|---|---|
| 1a | n-Pr | 4-chloro-3-trifluoro-methylphenyl | oil | 337 | 1735 $cm^{-1}$ (liq.film) | 0.80-2.20, 7H, m; 4.60, 6H,m, 7.40, 1H, d, 7; 8.10,7H,m |
| 2a | n-Pr | 4-bromo-3-chlorophenyl | oil | | 1720 $cm^{-1}$ (S)(liq.film) | 0.80-2.00, 7H, m; 4.40, 6H, m; 7.60, 2H, m; 7.95, 1H,m |
| 3a | n-Pr | 4-bromo-3,5-dichlorophenyl | | 381,383 | | 0.80-2.00, 7H, m; 4.20-4.50, 6H, m; 7.80, 2H, m |
| 4a | n-Pr | 3,5-di-chloro-phenyl | | 303 | | 0.80-2.00, 7H, m; 4.60, 6H, m; 7.50, 1H, m, 7.90, 2H,m |
| 5a | n-Pr | 3,5-di-trifluoro-methylphenyl | | 371 | | 0.80-2.00, 7H, m; 4.60, 6H, m; 8.10, 1H,m; 8.50, 2H,m |
| 6a | n-Pr | 4-amino-3,5-dichlorophenyl | | | | 0.80-2.00, 7H, m; 4.60, 6H,m; 5.10, 2H broad signal; 8.00, 2H,s |

TABLE III (Continued)

| | | | | | |
|---|---|---|---|---|---|
| 7a | n-Bu | 4-chloro-3-trifluoro-methylphenyl | oil | | 0.80-2.00, 9H, m; 4.60, 6H, m; 7.60, 1H, d, 6; 8.20,1H,m 8.40, 1H, m |
| 8a | n-Pr | 4-bromo-2,3,5,6-tetrafluorophenyl | | | 0.95, 3H, t; 1.3, 2H, m; 1.75, 2H, m; 4.45, 4H, q; 4.55, 2H, s |
| 9a | n-Pr | 4-chloro-3-iodo phenyl | oil | 395 | 0.80-2.00, 7H, m; 4.40, 6H,m; 7.20, 1H, d, 7; 7.60, 1H, dd, 7 and 1.5; 8.20, 1H, d, 1.5 |
| 13a | n-Pr | pentafluoro-phenyl | oil | 325 | 0.80-2.00, 7H, m; 4.50, 6H, m |
| 14a | n-Pr | 3,4-di-iodo-phenyl | oil | 487 | 0.95, 3H, t, 7; 1.30, 2H, m; 1.80, 2H, m; 4.55,6H, m; 7.60, 1H, dd, 7 and 1.5; 7.95,2H,d, 7; 8.50, 1H, d, 1.5 |
| 15a | t-Bu | 3-cyano-4-methylphenyl | | | 1.05,9H,s; 2.60,3H,s; 4.45,2H,s; 4.55,4H,q; 7.4,1H,d; 8.30,1H,d |
| 16a | t-Bu | 3,4,5-trichloro-phenyl | | | 1.00,9H,s;4.40,2H,s;4.50,4H,q; 7.95,2H,s |
| 17a | t-Bu | 3-methoxy-4-chlorophenyl | | | 1.00,9H,s; 3.90,3H,s;4.40,2H,s; 4.55,4H,q; 7.4,1H,d; 7.6,2H,m |
| 18a | t-Bu | 4-iodo-3,5-dichlorophenyl | | | 1.05,9H,s; 4.45,2H,s; 4.55,4H,q; 7.95,2H,s |
| 19a | t-Bu | 3-cyano-4-chlorophenyl | | | 1.05,9H,s; 4.50,2H,s; 4.60,4H,q; 7.6,1H,d; 8.25,1H,d of d; 8.35,1H,d |

TABLE III (Continued)

| | | | | |
|---|---|---|---|---|
| 20a | t-Bu | 3-cyano-4-methoxyphenyl | | 1.05,9H,s; 4.0,3H,s; 4.45,2H,s; 4.55,4H,q; 7.05,1H,d; 8.30 |
| 21a | t-Bu | 4-azido-3,5 dichlorophenyl | | 1.05,9H,s; 4.45,2H,s; 4.55,4H,q; 7.95,2H,s |
| 22a | t-Bu | 4-cyano-3,5 dichlorophenyl | | 1.05,9H,s; 4.50,2H,s; 4.60,4H,q; 8.10,2H,s |
| 23a | t-Bu | 4-bromo-3,5-dichlorophenyl | | 1.05,9H,s; 4.40,2H,s;4.55,4H,q; 8.0,2H,s |
| 24a | t-Bu | 3,4,5-triiodo phenyl | | 1.05,9H,s; 4.45,2H,s; 4.55,4H,q; 8.40,2H,s |
| 25a | t-Bu | 2-chloro-4-cyanophenyl | | 1.05,9H,s; 4.45,2H,s; 4.60,4H,q; 7.6,1H,d of d; 7.75,1H,d |
| 27a | t-Bu | 3,4-difluorophenyl | | 1.05,9H,s; 4.45,2H,s; 4.60,4H,q; 7.25,1H,m; 7.8,2H,m |
| 28a | n-Pr | 3-fluoro-4-iodophenyl | oil | 1.00,3H,t,7; 1.20-2.00,4H,m; 4.45,6H; 7.30-7.90,3H,m. |

The benzoic acid precursors required in the syntheses of the new compounds were obtained as follows:- 3,5-Dichlorobenzoyl chloride (used in synthesis of Compound 4) and 3,4-difluorobenzoyl chloride (used in

the synthesis of Compound 27), was supplied by Aldrich and 3,5-di-trifluoromethylbenzoyl chloride (used for Compound 5) was supplied by Fluorochem.

Synthesis of 4-bromo-3-chlorobenzoic acid (used in synthesis of Compound 2)

i) A solution of methyl 4-aminobenzoate (15 g supplied by Aldrich) in acetonitrile was heated at 0° with stirring. N-chlorosuccinimide (14.6 g) was added in one portion and the reaction mixture was refluxed, with stirring, for 4 hours. The reaction mixture was cooled and the solvent was removed in vacuo. The residue was taken up in dichloromethane and the extracts were washed with cold 1M aqueous sodium hydroxide solution. The dichloromethane solution was dried over anhydrous magnesium sulphate and the solvent was removed in vacuo. Methyl 4-amino-3-chloro benzoate (17.1 g) (synthesis 1985, 669-670) was obtained as a crystalline solid and was used without further purification.

Gas-liquid chromatography (g.l.c.): OV-210 at 150° produced one peak.

Nuclear magnetic resonance spectrum (N.M.R.) was as follows: $^1H$ (p.p.m. from TMS in $CDCl_3$, integral, number of peaks, $J_{Hz}$): 3.85, 3H, s; 4.60, 2H, broad signal; 6.70, 1H, d, 7; 7.80, 2H, m.

ii) A solution of methyl 4-amino-3-chlorobenzoate (17.0 g) in 48% hydrobromic acid (200 ml) was stirred at 0°. Sodium nitrite solution (6.3 g in 25 ml of water) was added dropwise over 45 minutes and the reaction mixture was maintained at 0-5°C. The mixture was stirred at 0-5° for 2 hours. A solution of sodium metabisulphite (5.5 g) and sodium hydroxide (3.6 g) in water (40 ml) was added to a hot solution of copper (II) sulphate pentahydrate (25.5 g) and sodium bromide (12.3 g) in water (80 ml). The stirred suspension was maintained at 75° and the cold diazonium salt solution was added. The mixture was stirred for 15 minutes and 48% hydrobromic acid (30 ml) was added. The mixture was cooled to room temperature and extracted with diethyl ether. The ethereal extracts were washed with water and brine and dried over anhydrous magnesium sulphate. The solution was evaporated in vacuo. Methyl 4-bromo-3-chlorobenzoate (20.4 g) was obtained as a crystalline solid and was used without further purification.

Gas-liquid chromatography (g.l.c.): OV-210 at 180° produced one peak.

Nuclear magnetic resonance spectrum (N.M.R.) was as follows: $^1H$ (p.p.m. from TMS in $CDCl_3$, integral, number of peaks, $J_{Hz}$): 3.80, 3H, s; 7.50, 2H, m; 7.90, 1H, m.

iii) Methyl 4-bromo-3-chlorobenzoate (20 g) was refluxed, with stirring, in aqueous sodium hydroxide solution (60 ml of 10% solution) for 2 hours. The resulting solution was cooled, diluted with water and acidified with concentrated hydrochloric acid solution. The aqueous mixture was extracted with diethyl ether and the ethereal extracts were dried over anhydrous magnesium sulphate. The solvent was removed in vacuo 4-Bromo-3-chlorobenzoic acid (18 g) was obtained as an off-white solid and was used without further purification.

Nuclear magnetic resonance spectrum (N.M.R.) was as follows: $^1H$ (p.p.m. from TMS in $CDCl_3$, integral, number of peaks, $J_{Hz}$): 7.80,2H, m; 8.10, 1H, m; 10.30, 1H, broad signal.

Infrared spectrum (1R) (nujol mull): max 2650 (strong and broad), 1680 (s), 930 $cm^{-1}$ (m).

Synthesis of 4-bromo-3,5-dichlorobenzoic acid (used in synthesis of Compounds 3 and 23)

i) Sulphuryl chloride (30 ml) was added over 30 minutes to a stirred solution of methyl 4-aminobenzoate (25 g) in dry chloroform (250 ml), at 0°. The reaction mixture was then refluxed, with stirring, for 6 hours. The reaction mixture was cooled and poured into a mixture of ice and water. The mixture was basified with 2N sodium hydroxide solution. The mixture was then extracted with ethyl acetate. The ethyl acetate extracts were dried over anhydrous magnesium sulphate and the solvent was removed in vacuo. Methyl 4-amino-3,5-dichlorobenzoate was obtained as a dark solid (32 g).

Gas-liquid chromatography (g.l.c.): OV-101 at 200° produced one peak.

Nuclear magnetic resonance spectrum (N.M.R.) was as follows: $^1H$ (p.p.m. from TMS in $CDCl_3/(CD_3)_2SO$, integral, number of peaks): 3.70, 3H, s; 5.10, 2H, broad signal, 7.60, 2H, s.

ii) Methyl 4-bromo-3,5-dichlorobenzoate was prepared from 4-amino-3,5-dichlorobenzoate in the same manner as described for the synthesis of methyl 4-bromo-3-chlorobenzoate. Methyl 4-bromo-3,5-dichlorobenzoate was obtained as a dark solid.

Gas-liquid chromatography (g.l.c.): OV-101 at 200° produced one peak.

Nuclear magnetic resonance spectrum (n.M.R.) was as follows: $^1H$ (p.p.m. from TMS in $CDCl_3$, integral, number of peaks): 3.95, 3H, s; 8.00, 2H, s.

iii) 4-Bromo-3,5-dichlorobenzoic acid was prepared from methyl 4-bromo-3,5-dichlorobenzoate in the same manner as described for the synthesis of 4-bromo-3-chlorobenzoic acid.

Synthesis of 4-amino-3,5-dichlorobenzoic acid (used in synthesis of Compound 6)

4-Amino-3,5-dichlorobenzoic acid was prepared from 4-aminobenzoic acid in a similar manner to that described for the synthesis of methyl 4-amino-3,5-dichlorobenzoate from methyl 4-aminobenzoate.

4-Amino-3,5-dichlorobenzoic acid was obtained as a colourless solid (mpt 290° recrystallised from ethanol).

Nuclear magnetic resonance spectrum (N.M.R.) was as follows: $^1H$ (p.p.m. from TMs in $(CD_3)_2SO$, integral, number of peaks): 7.50, 3H, broad signal; 7.80, 2H s.

Synthesis of 3-fluoro-4-iodobenzoic acid (used in synthesis of compound 28)

3-Fluoro-4-iodobenzoic acid was prepared by the method described in A.S.Tomcufcik, D.R.Seeger, J.Org.Chem, 1961, 26, 3351.

Synthesis of 4-bromo-2,3,5,6-tetrafluorobenzoic acid (used in the synthesis of Compound 8)

4-Amino-2,3,5,6-tetrafluorobenzoic acid (4.0 g, supplied by Aldrich) was added slowly to a stirred solution of anhydrous copper (II) bromide (5.32 g) and tert-butyl nitrite (3.1 g) in anhydrous acetonitrile (80 ml), at 65°C, under nitrogen.

After 20 minutes nitrogen ceased to be evolved and the cooled mixture was poured into dilute hydrochloric acid and the aqueous mixture was extracted with diethyl ether. The ethereal extracts were washed with dilute hydrochloric acid and dried over anhydrous magnesium sulphate. The solvent was removed in vacuo and 5.2 g of the acid was obtained. The acid was identified as its methyl ester.

Synthesis of 3,4,5-trichlorobenzoic acid (used in the synthesis of Compound 16)

3,4,5-Trichlorobenzoic acid was prepared from 4-amino-3,5-dichlorobenzoic acid, copper (II) chloride and tert-butyl nitrite in acetonitrile using the methodology used in the synthesis of 4-bromo-2,3,5,6-tetrafluorobenzoic acid.

Nuclear magnetic resonance spectrum (NMR) was as follows: 'H (ppm from TMS in $(CD_3)_2CO$, integral, number of peaks): 8.05,2H,s.

Mass spectrum $M^+$ 224.

Synthesis of 4-chloro-3-methoxybenzoic acid (used in the synthesis of Compound 17)

4-Chloro-3-methoxybenzoic acid was prepared from 4-amino-3-methoxybenzoic acid (supplied by Biochemical Laboratories Inc) using the methodology used in the preparation of 3,4,5-trichlorobenzoic acid.

Nuclear magnetic resonance spectrum (NMR) was as follows: 'H (ppm from TMS in $(CD_3)_2CO$, integral, number of peaks):

3.95, 3H, s; 7.50, 1H, d; 7.60, 1H, dd; 7.65, 1H, d.

Synthesis of 4-cyano-3,5-dichlorobenzoic acid (used in the synthesis of Compound 22)

(i) Sodium nitrite (20 g) in water (50 ml) was added to a mixture of methyl 4-amino-3,5-dichlorobenzoate (8.6 g) in concentrated sulphuric acid (110 ml) and water (95 ml) at 0°C. The mixture was stirred until all the solid dissolved (overnight at 0°). The solution was added slowly to a stirred mixture of copper (I) cyanide (7.1 g), potassium cyanide (21.7 g) and potassium carbonate (285 g) in water (250 ml) and benzene (175 ml). The temperature during the addition was kept below 15°C. The temperature was maintained at 100°C for 90 minutes. The mixture was cooled. The solid was filtered off and washed with benzene. The organic layer was separated from the aqueous layer, dried over anhydrous magnesium sulphate and the solvent removed in vacuo leaving methyl 4-cyano-3,5-dichlorobenzoate (6.78 g).

Nuclear magnetic resonance spectrum (NMR) was as follows: 'H (ppm from TMS in $CDCl_3$, integral, number of peaks):

3.95, 3H, s; 8.05, 2H, s.

Mass spectrum $M^+$ 229.

(ii) To a solution of methyl 4-cyano-3,5-dichlorobenzoate (5.4 g) in methanol (150 ml) was added a solution of sodium hydroxide (5.0 g) in water (100 ml) and methanol (100 ml) at 0°C. The mixture was stirred for 2 hours and acidified with dilute hydrochloric acid. The methanol was removed in vacuo and the resulting aqueous mixture was extracted with diethyl ether. The ethereal extracts were dried over anhydrous magnesium sulphate and evaporated in vacuo to give 4-cyano-3,5-dichlorobenzoic acid (4.1 g).

Nuclear magnetic resonance spectrum (NMR) was as follows: 'H (ppm from TMS in $(CD_3)_2CO$, integral, number of peaks):

8.15, 2H, s.

Synthesis of 3,5-dichloro-4-iodobenzoic acid (used in the synthesis of Compound 18)

(i) Sodium nitrite (20 g) in water (50 ml) was added to a stirred mixture of methyl 4-amino-3,5-dichlorobenzoate (6.23 g) in concentrated sulphuric acid (110 g) and water (95 ml) at 0°C. The mixture was stirred until all the solid had dissolved (overnight at 0°C). To the stirred solution was added a solution of potassium iodide (25 g) in water at 50°C and the resulting mixture was stirred at 20°C overnight. The mixture was extracted with diethyl ether. The ethereal extracts were washed with aqueous sodium bisulphite solution and then dried over anhydrous magnesium sulphate. The solvent was evaporated in vacuo and the residue was chromatographed on silica, eluting with hexane: dichloromethane 7:3. 3.75 g of methyl 3,5-dichloro-4-iodobenzoate was obtained.

Nuclear magnetic resonance spectrum (NMR) was as follows: 'H (ppm from TMS in $CDCl_3$, integral, number of peaks):

3.90, 3H, s; 7.95, 2H, s.

Mass spectrum $M^{+1}$ 331.

(ii) To a solution of sodium hydroxide (3.0 g) in water (60 ml) and methanol (60 ml) was added a solution of methyl 3,5-dichloro-4-iodobenzoate (3.75 g) in methanol (50 ml) at 0°C. The mixture was stirred at 20°C for 3

days.

The methanol was removed in vacuo and the aqueous mixture was extracted with diethyl ether. The aqueous layer was acidified with dilute hydrochloric acid. The aqueous mixture was extracted with diethyl ether and the ethereal extracts were dried over anhydrous magnesium sulphate. The solvent was removed in vacuo and 3,5-dichloro-4-iodobenzoic acid (3.4 g) was obtained.

Nuclear magnetic resonance spectrum (NMR) was as follows: 'H (ppm from TMS in $(CD_3)_2CO$, integral, number of peaks):

7.95, 2H, s.

Synthesis of 3-cyano-4-chlorobenzoic acid (used in the synthesis of Compound 19)

Sodium nitrite (10 g) was added to a stirred mixture of 3-amino-4-chlorobenzoic acid (10 g, supplied by Aldrich) in concentrated sulphuric acid (110 g) and water (95 ml) at 0°C. The solution was stirred at 0°C for 2 hours and was added slowly to a stirred mixture of copper (I) cyanide (7.1 g), potassium cyanide (21.7 g) and potassium carbonate (285 g) in water (250 ml) and benzene (175 ml). The temperature during the addition was kept below 15°C. After the addition was complete the mixture was maintained at 80°C for 1 hour (more benzene was added to prevent foaming). The mixture was cooled, filtered and the solid was washed with benzene and diethyl ether. The aqueous layer was separated, acidified with hydrochloric acid and extracted with diethyl ether. The ethereal extracts were dried over anhydrous magnesium sulphate and evaporated in vacuo. The residue was chromatographed on silica, eluting with hexane: ethyl acetate 3:2. 2.1 g of 3-cyano-4-chlorobenzoic acid was obtained.

Nuclear magnetic resonance spectrum (NMR) was as follows: 'H (ppm from TMS in $(CD_3)_2CO$ , integral, number of peaks):

7.85, 1H, d; 8.30, 1H, dd; 8.40, 1H, d.

Synthesis of 3-cyano-4-methoxybenzoic acid (used in the synthesis of Compound 20)

3-Cyano-4-methoxybenzoic acid was prepared from 3-amino-4-methoxybenzoic acid (supplied by Aldrich) using the methodology used in the synthesis of 3-cyano-4-chlorobenzoic acid.

Nuclear magnetic resonance spectrum (NMR) was as follows: 'H (ppm from TMS in $(CD_3)_2CO$, integral, number of peaks):

4.05, 3H, s; 7.3, 1H, d; 8.25, 2H, m.

Synthesis of 2-chloro-4-cyanobenzoic acid (used in the synthesis of Compound 25)

To a stirred suspension of 4-amino-2-chlorobenzoic acid (10 g, supplied by Aldrich) in water (150 ml) and concentrated sulphuric acid (20 ml) at -2°C was added a solution of sodium nitrite (4.8 g) in water (75 ml) at -2°C. After the solid had dissolved, urea was added to destroy excess nitrous acid. The resulting solution was added to a stirred solution of copper (I) cyanide (9.0 g) and potassium cyanide (13.0 g) in water (30 ml) at 60-70°C and the mixture was heated to 85-90°C for 1 hour. The hot mixture was filtered. The filtrates were cooled and the product was filtered off. 1.3 g of 2-chloro-4-cyanobenzoic acid was obtained.

Nuclear magnetic resonance spectrum (NMR) was as follows: 'H (ppm from TMS in $(CD_3)_2CO$, integral, number of peaks):

7.85, 1H, dd; 8.0, 1H, d; 8.05 1H, d.

Mass spectrum M+1 182.

Synthesis of 4-azido-3,5-dichlorobenzoic acid (used in the synthesis of Compound 21)

To a stirred solution of 4-amino-3,5-dichlorobenzoic acid (10 g) in glacial acetic acid (100 ml) at 0°C was added a solution of sodium nitrite (4.2 g) in concentrated sulphuric acid (acid 20 ml) at 0°C. The temperature was kept at below 5°C throughout the addition. After stirring for 30 minutes, water (100 ml) was added dropwise keeping the temperature below 10°C. Urea was then added to decompose excess nitrous acid. A solution of sodium azide (6.5 g) in water (60 ml) was added. After 30 minutes the solution was allowed to warm to 20°C and the solid was filtered off, washed with water, dried and recrystallized from ethanol. 7.0 g of 4-azido-3,5-dichlorobenzoic acid was obtained.

Nuclear magnetic resonance spectrum (NMR) was as follows: 'H (ppm from TMS in $(CD_3)_2CO$, integral, number of peaks):

7.95, 2H, s.

Mass spectrum M+1 232.

The intermediate benzoic acid, acid chlorides and methyl esters were characterised by the data set out in Table IV below.

TABLE IV

Characterising Data for Benzoic Acid Derivatives

| Compound | Mass Spectrum Chemical Ionisation | Nuclear Magnetic Resonance Spectra (in $CDCl_3$ unless otherwise stated) |
|---|---|---|
| 4-Azido 3,5-dichlorobenzoic acid | 232 | in $(CD_3)_2CO$ 7.95,2H,s |
| 3,4,5-trichloro-benzoic acid | 224 | in $(CD_3)_2CO$ 8.05,2H,s |
| 4-amino-3,5-dichlorobenzoyl chloride | 224 | 5.2,2H,broad s; 7.95,2H,s |
| Methyl 4-cyano-3,5-dichlorobenzoate | 229 | 3.95,3H,s; 8.05,2H,s |
| 4-cyano-3,5-dichlorobenzoac acid | 215 | in $(CD_3)CO$ 8.15,2H,s |
| 4-cyano-3,5-dichlorobenzoyl chloride | - | 8.15,2H,s |
| Methyl-4-iodo-3,5 dichlorobenzoate | 331 | 3.90,3H,s; 7.95,2H,s |
| 4-Iodo-3,5-dichlorobenzoic acid | - | in $(CD_3)_2CO$ 7.95,2H,s |
| 4-Iodo-3,5-dichlorobenzoyl chloride | - | 8.00,2H,s |
| 3,4,5-trichloro-benzoyl chloride | - | 8.10,2H,s |
| 4-Bromo-3,5-dichlorobenzoyl chloride | - | 8.05,2H,s |
| 3,4,5-triodo-benzoyl chloride | - | 8.45,2H,s |

| | | |
|---|---|---|
| 3-cyano-4-methyl-benzoyl chloride | - | 2.65,3H,s; 7.45,1H,d; 8.20,1H,d of d; 8.90,1H,d |
| 4-chloro-3-methoxy-benzoic acid | - | in $(CD_3)_2CO$ 3.95,3H,s; 7.50,1H,d; 7.60,1H,d of d; 7.65,1H,d |
| 4-chloro-3-methoxy-benzoyl chloride | - | 3.95,3H,s; 7.50,1H,d; 7.55,1H,d; 7.80,1H,d of d |
| 4-chloro-3-cyano-benzoic acid | - | in $(CD_3)_2CO$ 7.85,1H,d; 8.30,1H,d of d; 8.40,1H,d |
| 4-chloro-3-cyano-benzoyl chloride | - | 7.7,1H,d; 8.25,1H,d of d; 8.40,1H,d |
| 4-methoxy-3-cyano benzoic acid | - | in $(CD_3)_2CO$ 4.05,3H,s; 7.3,1H,d; 8.25,2H,m |
| 4-methoxy-3-cyano-benzoyl chloride | - | 4.05,3H,s; 7.05,1H,d; 8.3,1H,d of d; 8.35,1H,d |
| 4-cyano-2-chloro-benzoic acid | 182 | in $(CD_3)_2CO$ 7.85,1H,d of d; 8.0,1H,d; 8.05,1H,d |
| 4-cyano-2-chloro-benzoyl chloride | - | 7.70,1H,d of d; 7.80,1H,d; 8.10,1H,d |
| 4-bromo-2,3-5,6-tetrafluorobenzoic acid | 289 | identified as methyl ester |
| 4-bromo-2,3,5,6-tetrafluorobenzoyl chloride. | 289 | identified as methyl ester |

EXAMPLE 2

1-(3-chloro-4-methoxyphenyl)-4-t-butyl-2,6,7-trixoabicyclo [2.2.2]octane

i) Sodium pellets (14g) were dissolved in dry methanol under an atmosphere of dry nitrogen. 3,4-Dichlorobenzotrichloride (26.5g) was added and the mixture was refluxed for 96 hours. The cooled solution was partitioned between diethyl ether and water and the ether layer was separated, washed with water, dried over anhydrous magnesium sulphate and evaporated in vacuo to leave an oil.

Nuclear magnetic resonance spectrum (N.M.R.) for methyl (3-chloro-4-methoxyortho-benzoate) was as follows:-

1H (ppm from TMS in $CDCl_3$, intergral, number of peaks) 3.10, 9H, s; 3.90, 3H, s; 6.90, 1H, d; 7.40, 1H, m; 7.55, 1H, m.

ii) 1-(3-chloro-4-methoxyphenyl)-4-t-butyl-2,6,7-trioxabicyclo [2.2.2] octane was prepared from 3-t-butyl-3-hydroxymethyl-propan-1,3-diol and trimethyl (3-chloro-4-methoxyorthobenzoate) using the method described in European Patent Application 86305820.0 (EP-A )

EXAMPLE 3

1-(4-chloro-3-ethynylphenyl)-4-n-propyl-2,6,7-trioxabicylco [2.2.2] octane

i) Bis-triphenylphosphine palladium dichloride (20mg) and cuprous chloride (5mg) were added to a stirred solution of 1-(4-chloro-3-iodophenyl)-4-n-propyl-2,6,7-trioxabicyclo [2.2.2] octane (1.0g) and trimethylsilylacteylene (0.54ml) in dry diethylamine (10mls). The mixture was stirred at room temperature for 12 hours. The mixture was evaporated in vacuo and the residue was dissolved in diethyl ether. The ethereal solution was washed with water, dried over anhydrous magnesium sulphate and evaporated in vacuo. The residue was purified by column chromatography on alumina eluting with 1:10 dichloromethane: hexane, saturated with ammonia. 4-n-propyl-1-{[4-chloro-3-(2-trimethylsilylethynyl) phenyl]}-2,6,7-trioxabicyclo [2.2.2] octane was obtained as a pale brown solid (0.8g).

ii) Tetrabutylammonium fluoride solution (2.2ml, IM solution in tetrahydrofuran) was added to a stirred solution of 4-n-propyl-1-{[4-chloro-3-(2-trimethylsilylethynyl)phenyl]}-2,6,7-trioxabicyclo[2.2.2] octane (0.65g) in dry tetrahydrofuran (10mls). The mixture was stirred for 2 hours at room temperature. The solvent was removed in vacuo and the residue was dissolved in dry diethyl ether. The ethereal solution was washed with water, dried over anyhydrous magnesium sulphate and evaporated in vacuo The residue was recrystallised from hexane containing a few drops of dichloromethane.
1-(4-chloro-3-ethynylphenyl)-4-n-propyl-2,6,7-trioxabicylco [2.2.2] octane was obtained as a colourless solid (0.35g).

EXAMPLE 4

1-(4-n-Butyl-2,3,5,6-tetrafluorophenyl)-4-n-propyl 2,6,7-trioxabicyclo [2.2.2]octane.

To a solution of 4-n-propyl-1-(pentafluorophenyl)-2,6,7-trioxabicyclo [2.2.2] octane (0.5g) in dry diethyl ether (50 ml) at -60°C, under a current of dry nitrogen, was added n-butyllithium (1.1ml, 1.6M in hexane). The reaction mixture was stirred at -30°C for 2 hours. Water was added and the aqueous mixture was extracted with diethyl ether. The ethanol extracts were dried over anhydrous magnesium sulphate and the solvent was removed in vacuo. The residue was chromatographed on alumina, eluting with 1:10 dichloromethane: hexane, saturated with ammonia. 1-(4-n-Butyl-2,3,5,6-tetrafluorophenyl)-4-n-propyl-2,6,7-trioxabicyclo [2.2.2] octane was obtained as a colourless solid (0.14g).

A. Lethal Activity Against Houseflies

The activity of compounds of the invention against unanaesthetised female Musca Domestica (WRL strain) was demonstrated by topical application to the test insect of a solution of the compound under test in butanone, either on its own or in conjunction with 6ug piperonyl butoxide.

The activity of the compounds under test were assessed at 15 mins, 1 day and 2 days.

The following compounds were active at less than 30ug/fly:

(1); (26); (17); (15); (25).

The following compounds were active at less than 1ug/fly, when applied in conjunction with the synergistic, piperonyl butoxide:

(4); (2); (22); (23); (19); (18); (20); (28).

B. Lethal Activity Against Sitophilus granarius

The activity of the commpounds of the invention against S. granarius adults was demonstrated by the addition of the compound in acetone solution to grain, to which the insects were later infected. Mortality was assessed after 6 days.

The following compounds gave activity at concentrations of less than 200 ppm in grain:
(4); (1); (2); (3); (26); (22); (19); (17).
The following compounds gave activity at concentrations of less than 50 ppm in grain:
(23); (18); (28).

C. The Lethal Activity Against Tetranychus urticae

The activity of the compounds of the invention against a mixed population of Tetranychus urticae was demonstrated by spray application to the mites on leaf discs of a water emulsion (94.5% water, 5% acetone, 0.5% surfactant).

The following compound was active at less than 5000 ppm solution.

(3).

The following compounds were active at less than 1000 ppm.

(7); (18).

$LD_{50}$ - data

Compounds in DMSO were administered orally to mice. The following compounds had an $LD_{50}$ in excess of 200 mg/kg 1, 2, 3, 4, 5, 7, 20.

The following compounds had an $LD_{50}$ in excess of 20 mg/kg.

(25), (26).

## Claims

1. A compound of the formula I

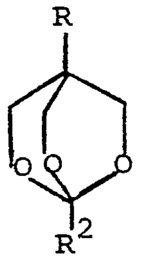

(I)

wherein R is $C_{2-10}$ alkyl, alkenyl or alkynyl, each optionally substituted by, or methyl-substituted by, cyano, halo, $C_{3-4}$ cycloalkyl, $C_{1-4}$ alkoxy, $C_{1-4}$ acyloxy, amino, optionally sustituted by one $C_{2-4}$ alkoxycarbonyl or $C_{1-4}$ acyl group or by one or two $C_{1-4}$ alkyl groups, or a group $S(0)_mR^4$ where $R^4$ is $C_{1-4}$ alkyl and m is 0, 1 or 2, or R is $C_{3-10}$ cycloalkyl, $C_{4-10}$ cycloalkenyl or phenyl, each optionally substituted by $C_{1-4}$ alkoxy, $C_{1-3}$ alkyl, $C_{2-4}$alkenyl, $C_{2-4}$ alkynyl, $C_{1-4}$ acyloxy, halo, cyano,amino optionally substituted by one or two $C_{1-4}$ alkoxyacyl, $C_{1-4}$acyl, $C_{1-4}$ acyloxy, $C_{1-4}$ alkyl or $C_{1-4}$ acyloxy optionally substituted by an amino group mono or di-substituted by $C_{1-4}$alkyl groups or mono-substituted by phenyl optionally substituted by one to five halogen atoms, a group $S(O)_mR^4$ as defined hereinbefore or a group $COR^5$ wherein $R^5$ is $C_{1-4}$alkyl, $C_{1-4}$ alkoxy or amino optionally substituted by one or two $C_{1-4}$ alkyl groups;

$R^2$ is a phenyl group substituted in at least two of the 3-, 4-, and/or 5-positions, position 4, when substituted, being substituted by halo; amino; nitro; azido; cyano; $C_{1-4}$ haloalkyl; $C_{1-4}$ alkyl; $C_{1-4}$ haloalkoxy; $C_{1-4}$alkoxy; $S(0)_mR^{4a}$, wherein $R^{4a}$ is a group $R^4$ as defined hereinbefore; or $C_{1-4}$ alkyl substituted by one to three fluorine atoms; or by a group $COR^5$ as defined hereinbefore; and at the 3- and/or 5-position at the phenyl ring when substituted, being substituted by a halo, $C_{1-4}$ haloalkyl, $C_{1-4}$ alkyl, $C_{1-4}$ haloalkoxy, $C_{1-4}$ alkoxy, $C_{1-4}$ alkyl substituted by one three fluorine atoms, $S(0)_mR^4$ as defined hereinbefore, nitro, azido or cyano group by a group $COR^5$ as defined hereinbefore or by a group of the formula $C{\equiv}CR^6$ wherein $R^6$ is hydrogen, or a silyl group substituted by three $C_{1-4}$ alkyl groups or two $C_{1-4}$ alkyl groups and a phenyl group; and further optionally substituted at the 2-and/or 6-position by fluoro, chloro except that:

1. when R is tert.butyl, then $R^2$ may not be 3,4-dichlorophenyl or pentafluorophenyl;
2. when R is n-propyl, then $R^2$ may not be 3-chloro-4-iodophenyl or 4-iodo-3-nitrophenyl;
3. when R is cyclohexyl, then $R^2$ may not be 3,4-dichlorophenyl;
4. when R is isopropyl, then $R^2$ may not be pentafluorophenyl.

2. A compound according to claim 1 wherein R is a propyl, butyl, pentyl, $C_{2-5}$ alkenyl or alkynyl, $C_{3-7}$cycloalkyl or phenyl group, the groups being unsubstituted or optionally substituted by chloro, bromo or fluoro.

3. A compound according to claim 1 wherein R is n-propyl, n-butyl, i-butyl, t-butyl or cycloalkyl.

4. A compound according to any one of the preceding claims wherein the phenyl group $R^2$ is substituted at positions 3 and 4 or at positions 3, 4 and 5.

5. A compound according to any one of the preceding claims wherein the phenyl group $R^2$ is substituted by fluoro, chloro, bromo, iodo, cyano, azido, $C_{1-3}$ alkyl or $C_{1-3}$ alkoxy each optionally substituted by halo, or $C_{2-3}$alkynyl optionally substituted by a silyl group substituted by three $C_{1-4}$ alkyl groups or two $C_{1-4}$ alkyl groups and a phenyl group.

6. A compound according to claim 1 which is

(a) 1-(3-cyano-4-methylphenyl)-4-tert.butyl-2,6,7-trioxabicyclo[2.2.2]octane,
(b) 1-(3,4,5-trichlorophenyl)-4-tert.butyl-2,6,7-trioxabicyclo[2.2.2]octane,
(c) 1-(3-methoxy-4-chlorophenyl)-4-tert.butyl-2,6,7-trioxabicyclo[2.2.2]octane,
(d) 1-(4-iodo-3,5-dichlorophenyl)-4-tert.butyl-2,6,7-trioxabicyclo[2.2.2]octane,
(e) 1-(3-cyano-4-chlorophenyl)-4-tert.butyl-2,6,7-trioxabicyclo[2.2.2]octane,
(f) 1-(3-cyano-4-methoxyphenyl)-4-tert.butyl-2,6,7- trioxabicyclo[2.2.2]octane,
(g) 1-(4-azido-3,5-dichlorophenyl)-4-tert.butyl-2,6,7-trioxabicyclo[2.2.2]octane,
(h) 1-(4-cyano-3,5-dichlorophenyl)-4-tert.butyl-2,6,7-trioxabicyclo[2.2.2]octane,
(i) 1-(4-bromo-3,5-dichlorophenyl)-4-tert.butyl-2,6,7-trioxabicyclo[2.2.2]octane,
(j) 1-(3,4,5-tri-iodophenyl)-4-tert.butyl-2,6,7-trioxabicyclo[2.2.2]octane,
(k) 1-(2-chloro-4-cyanophenyl)-4-tert.butyl-2,6,7-trioxabicyclo[2.2.2]octane,
(l) 1-(4-bromo-3-chlorophenyl)-4-n-propyl-2,6,7-trioxabicyclo[2.2.2]octane,
(m) 1-(4-bromo-3,5-dichlorophenyl)-4-n-propyl-2,6,7-trioxabicyclo[2.2.2]octane,

(n) 1-(4-chloro-3-trifluoromethylphenyl)-4-n-butyl-2,6,7-trioxabicyclo[2.2.2]octane or

(o) 1-(3-fluoro-4-iodophenyl)-4-n-propyl-2,6,7-trioxabicyclo[2.2.2]octane.

7. A process for preparing a compound of formula I as defined in any one of the preceding claims which comprises cyclisation, in the presence of an acid catalyst of a compound of formula

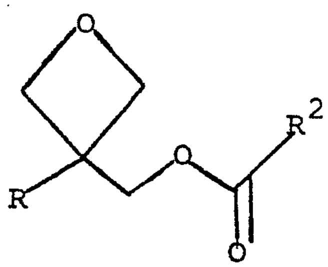

(II)

where R and $R^2$ are as defined in any one of the preceding claims.

8. A process according to claim 7 for the production of a compound in which the phenyl group $R^2$ contains a $-C\equiv C-R^6$ group, $R^6$ being as defined in claim 1, which comprises cyclising a compound of formula II in which the phenyl group $R^2$ contains an iodo group in the position eventually to be substituted by the $-C\equiv C-R^6$ group and reacting the iodophenyl compound with $HC\equiv C-R^6$.

9. A process according to claim 7 for the production of a compound in which the phenyl group $R^2$ contains an iodo substituent which comprises cyclising a compound of formula II in which the phenyl group $R^2$ contains a bromo substituent in the position eventually to be substituted by iodo, reacting the bromophenyl compound with an alkyl lithium and reacting the resulting lithium phenyl intermediate with iodine.

10. An insecticidal or acaricidal composition comprising a compound of formula I according to any one of claims 1 to 6 in admixture with a carrier or diluent.

11. A composition according to claim 10 comprising a synergist or potentiator for the formula I compound.

12. An insecticidal or acaricidal mixture comprising a compound of formula I as defined in any one of claims 1 to 6 in admixture with another pesticidal compound.

13. A method for the control of pests comprising application to the pest or to an environment susceptible to pest infestation of a compound according to any one of claims 1 to 6 or a composition or mixture according to any one of claims 10 to 12.

14. A compound of formula

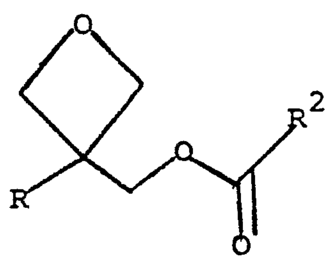

(II)

wherein R and $R^2$ are as defined in any one of claims 1 to 6.